# EUROPEAN PATENT APPLICATION

(11) **EP 3 306 300 A1**
(43) Date of publication of application: **11.04.2018**
(21) Application number: 15893352.3
(22) Date of filing: 28.05.2015
(51) Int. Cl.: G01N 5/00, D06H 3/00, G01G 19/00, G01N 33/36

(54) **HUMIDITY-DEPENDENT-MASS MEASUREMENT DEVICE AND HUMIDITY-DEPENDENT-MASS MEASUREMENT METHOD**

(71) Applicant: Kaken Test Center, Tokyo 103-0021 (JP)
(72) Inventor: IZUCHI Yukari, Tokyo 103-0021 (JP); KURAMOTO Kanya, Tokyo 103-0021 (JP)
(74) Representative: Rüger, Barthelt & Abel
(86) International application number: PCT/JP2015/065331
(87) International publication number: WO 2016/189718

(57) **Abstract**

A humidity-dependent-mass measurement device (1) includes: a thermostatic chamber (11) defining an interior space capable of containing a sample, having a through hole, formed in the ceiling and communicating between the interior space and outside, such that an atmosphere of the interior is maintained at a determined temperature and humidity; a balance (12) supported above the thermostatic chamber (11); a measurement tool for suspension from a weighing pan supporter of the balance (12), insertion through the through hole of the thermostatic chamber (11) without contacting an inner circumferential surface of the through hole, and holding the sample in the interior of the thermostatic chamber (11); a cover box (13) including an air inlet port and an air outlet port in a side surface, the cover box (13) containing therein a portion of the measurement tool between the thermostatic chamber (11) and the balance (12) in a state such that the measurement tool is suspended from the weighing pan supporter without contacting the cover box (13); and a dry air supplier (14) for causing dry air at or below a specified humidity to flow into the cover box (13) from the inlet port.

## Description

### Technical Field

The present disclosure relates to a humidity-dependent-mass measurement device and a humidity-dependent-mass measurement method that are used for measuring a mass of a substance for which the mass changes in a humidity-dependent manner.

### Background Art

Clothing is developed that has increased hygroscopicity and/or quick-drying performance, and testing is used for measurement of properties related to such moisture absorption and desorption, that is, such moisture absorption-desorption properties.

Patent Literature 1 describes moisture absorption-desorption coefficients of a fiber. Patent Literature 2 describes a moisture absorption parameter and a moisture desorption parameter of a measurement sample. Patent Literature 3 to Patent Literature 6 describe moisture absorption rates of fiber samples. Patent Literature 7 describes moisture absorption-desorption performance of a sample.

Patent Literature 8 discloses a measurement device for measurement of heat and moisture transfer characteristics of a sample. Patent Literature 9 discloses a testing device for measurement of moisture absorption-desorption characteristics of a test piece.

### Citation List

### Patent Literature

Patent Literature 1: Unexamined Japanese Patent Application Kokai Publication No. H09-41204
Patent Literature 2: Unexamined Japanese Patent Application Kokai Publication No. 2000-204230
Patent Literature 3: Unexamined Japanese Patent Application Kokai Publication No. 2000-314082
Patent Literature 4: Unexamined Japanese Patent Application Kokai Publication No. 2000-73234
Patent Literature 5: Unexamined Japanese Patent Application Kokai Publication No. 2001-146678
Patent Literature 6: Unexamined Japanese Patent Application Kokai Publication No. 2001-30402
Patent Literature 7: Unexamined Japanese Patent Application Kokai Publication No. 2001-172826
Patent Literature 8: Unexamined Japanese Patent Application Kokai Publication No. H10-18172
Patent Literature 9: Unexamined Japanese Patent Application Kokai Publication No. 2004-157086

### Summary of Invention

### Technical Problem

Feel concerning dampness is important for comfort when wearing clothing, and rather than just the amount of moisture interposed between the human body and the clothing, the speed of transfer of moisture, that is, the speeds of moisture absorption and desorption of the clothing are important factors in such feel. In conventional testing for measurement of moisture absorption-desorption performance, the mass of the sample is measured after the sample reaches equilibrium, and thus although the amount of moisture absorption or desorption can be measured in the equilibrium state, the speed of moisture absorption or desorption cannot be measured.

The devices described in Patent Literature 8 and Patent Literature 9 measure temperature and humidity of the surrounding air and estimate the moisture absorption amount or moisture desorption amount of the sample on the basis of differences in temperature and humidity between both sides of a sample. The moisture absorption amount or moisture desorption amount of the sample is not measured directly, and thus the moisture absorption amount or moisture desorption amount is not accurately known.

Resolution capability ratios of temperature and humidity measurements are lower than for mass measurement, and the error ratios thereof are larger than the error ratio for mass measurement. When the sample and the mass measurement device are placed in a thermostatic chamber, volume of the thermostatic chamber is high, and changing humidity fast enough to be capable of measuring the effect on the sample while maintaining uniform temperature and humidity conditions within the thermostatic chamber is difficult. Further, the mass measurement device is inherently to be used for measurement under fixed temperature and humidity conditions.

The present disclosure is developed in consideration of the aforementioned circumstances, and an objective of the present disclosure is to measure the mass of a sample varying in mass due to moisture in the surrounding air, the measurement being made so as to enable analysis of speed of moisture absorption and desorption of the sample.

### Solution to Problem

In order to attain the aforementioned objective, the humidity-dependent-mass measurement device according to the present disclosure includes:
a thermostatic chamber, defining a space in an interior thereof for containing a sample and including a ceiling having a through hole formed therein communicating between the space of the interior and an exterior of the thermostatic chamber, for maintaining an atmosphere of the interior at a determined temperature and humidity;
a balance supported above the thermostatic chamber;
a measurement tool for suspension from a weighing pan supporter, insertion through the through hole of the thermostatic chamber without contacting an inner circumferential surface of the through hole, and holding the sample in the interior of the thermostatic chamber;
a cover box including an air inlet port and an air outlet port in a side surface, the cover box containing therein a portion of the measurement tool between the thermostatic chamber and the balance in a state suspending the measurement tool from the weighing pan supporter without contacting the cover box; and
a dry air supplier for causing dry air at or below a specified humidity to flow into the cover box from the inlet port.

The humidity-dependent-mass measurement method according to the present disclosure uses a humidity-dependent-mass measurement device including: (i) a thermostatic chamber, defining a space in an interior thereof for containing a sample and including a ceiling having a through hole formed therein communicating between the space of the interior and an exterior of the thermostatic chamber, for maintaining an atmosphere of the interior at a determined temperature and humidity, (ii) a balance supported above the thermostatic chamber, (iii) a measurement tool for suspension from a weighing pan supporter, insertion through the through hole of the thermostatic chamber without contacting an inner circumferential surface of the through hole, and holding the sample in the interior of the thermostatic chamber, (iv) a cover box including an air inlet port and an air outlet port in a side surface, the cover box containing therein a portion of the measurement tool between the thermostatic chamber and the balance in a state suspending the measurement tool from the weighing pan supporter without contacting the cover box, and (v) a dry air supplier for causing dry air at or below a specified humidity to flow into the cover box from the inlet port. The method includes:
causing the sample to be held by the measurement tool within the interior of the thermostatic chamber;
maintaining the atmosphere of the interior of the thermostatic chamber at a determined temperature and a determined humidity; and
measuring a mass of the sample by the balance in a state in which the dry air at or below the specified humidity is made to flow from the inlet port of the cover box into the cover box.

### Advantageous Effects of Invention

The present disclosure enables measurement of the mass of the sample for which the mass varies in accordance with humidity of the atmosphere, such that speeds of moisture absorption and desorption of the sample can be measured.

### Brief Description of Drawings

FIG. 1 is a block diagram illustrating an example configuration of a humidity-dependent-mass measurement device according to an embodiment of the present disclosure;
FIG. 2 is a top view and a side view illustrating an example of a cover box according to the embodiment;
FIG. 3 is a front view illustrating an interior of a thermostatic chamber according to the embodiment;
FIG. 4 is a drawing illustrating an example of a measurement tool according to the embodiment;
FIG. 5 is a top view illustrating an example of a holder fixing a sample according to the embodiment;
FIG. 6 is a graph illustrating results of weighing with and without providing of the cover box according to the embodiment;
FIG. 7 in graphs illustrates tracking ability and reproducibility of results of measurement, with respect to changes of humidity, of moisture absorption and desorption amounts measured by the humidity-dependent-mass measurement device according to the embodiment;
FIG. 8 in graphs illustrates reproducibility of results of measurement of the moisture absorption and desorption amounts measured by the humidity-dependent-mass measurement device according to the embodiment; and
FIG. 9 is a table illustrating time-wise changes of a moisture absorption speed and a moisture desorption speed according to the embodiment.

### Description of Embodiments

Embodiments of the present description are described below with reference to figures. In the figures, components that are the same or equivalent are assigned the same reference signs.

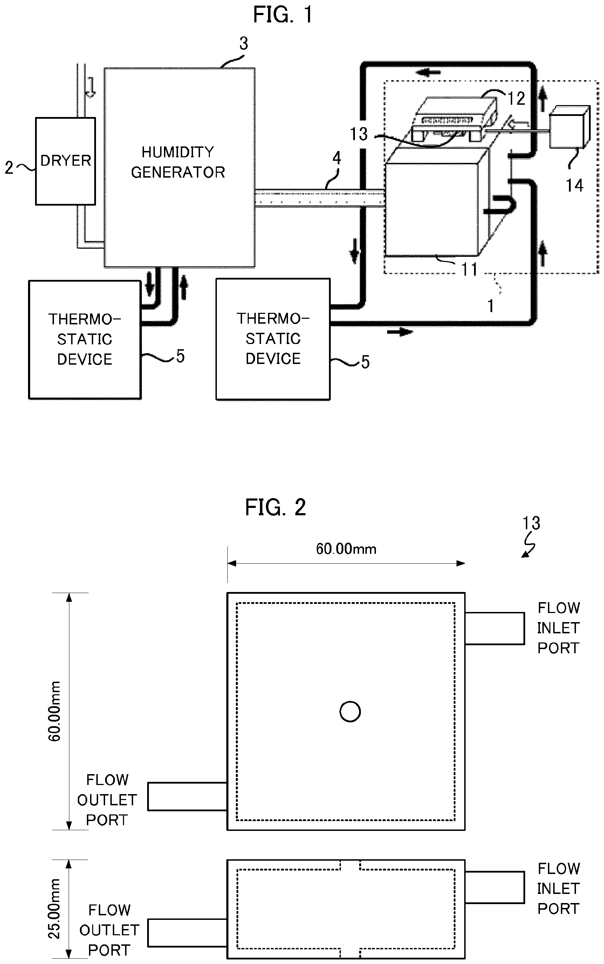

FIG. 1 is a block diagram illustrating an example configuration of a humidity-dependent-mass measurement device according to an embodiment of the present disclosure. The humidity-dependent-mass measurement device 1 includes a thermostatic chamber 11, a balance 12, a cover box 13, and a dry air supplier 14. In the example of FIG. 1, compressed air is dried by a dryer 2 and is supplied to a humidity generator 3. The humidity generator 3 controls humidity of the supplied air at a set humidity by the divided flow method, and supplies the humidity-controlled air to the thermostatic chamber 11 via a condensation-preventing heat-retention tube 4. The air within the humidity generator 3 and the air within the thermostatic chamber 11 are controlled at a set temperature by circulation of thermostatic water of the thermostatic device 5. The thermostatic chamber 11 has a water-circulating double wall structure and is capable of maintaining constant temperature.

Volume of the thermostatic chamber 11 enables stabilization of temperature and humidity in a short time period of, for example, 10 minutes. Thus the interior of the thermostatic chamber 11 in a short time period can be stabilized in the set thermo-hydrostatic state. The method of producing a thermo-hydrostatic state at set values in the interior of the thermostatic chamber 11 is not limited to the method described here, and any existing technique may be used. Here, the humidity range to which humidity is capable of being set by using the humidity generator 3 is taken to be 5% RH to 95% RH, and accuracy is taken to be ±0.8% RH in an environment at 23°C. The temperature range that is capable of being set using the thermostatic device 5 is taken to be -10°C to 80°C, and accuracy is taken to be ±0.10°C.

The humidity-dependent-mass measurement device 1 includes a measurement tool (not illustrated) for setting the sample in the interior of the thermostatic chamber 11. The measurement tool includes a suspension tool for suspension from a weighing pan supporter of the balance 12, and a holder detachably attached to the suspension tool in the interior of the thermostatic chamber 11. Through holes for passing the suspension tool are arranged in the ceiling of the thermostatic chamber 11 and in the ceiling and bottom of the cover box 13. The suspension tool suspended from the weighing pan supporter of the balance 12 is inserted in the through holes of the cover box 13 and the thermostatic chamber 11 without contacting the inner circumferential surfaces of the through holes. When the user fixes the sample to the holder and attaches the holder to the suspension tool, the sample can be suspended without contacting the interior walls of the thermostatic chamber 11. Mass of the sample is measured by the balance 12 and changes due to changes of temperature and humidity of the interior of the thermostatic chamber 11.

The dry air supplier 14 generates dry air having a humidity less than or equal to a specified humidity, and supplies the dry air to the cover box 13. The interior of the cover box 13 is hollow, and the dry air generated by the dry air supplier 14 is supplied from an inlet port and vents from an outlet port. The air within the thermostatic chamber 11 is thus prevented from rising toward the balance 12, and condensation is prevented. The dry air generated by the dry air supplier 14 may be set to the same temperature as the interior of the thermostatic chamber 11. Further, the cover box 13 may be provided in the interior of the thermostatic chamber 11 so as to contact the ceiling of the thermostatic chamber 11.

FIG. 2 is a top view and a side view illustrating an example of the cover box according to the embodiment. In the example of FIG. 2, the cover box 13 has a width of 60.00 mm, a length of 60.00 mm, and a height of 25.00 mm, and in a center of a horizontal surface, is equipped with a through hole for passing the suspension tool. The inlet port and the outlet port are disposed diagonally opposite to each other, so that the direction of inflow of the dry air is displaced from a direction of a straight line interconnecting the inlet port and the suspension tool and is displaced from a direction of a straight line interconnecting the inlet port and the outlet port. Due to such inflow, the dry air passes through the vicinity of the through hole through which the suspension tool passes, and the inflowing dry air does not directly contact the suspension tool, thereby enabling suppression of the effect of momentum of the inflowing dry air on the suspension tool. Further, the inlet port is arranged at a position higher than the outlet port to allow the dry air to easily flow through the interior of the cover box 13.

Further, even in the case in which the direction of inflow of the dry air matches the direction of the straight line interconnecting the inlet port and the suspension tool, for example, the inflowing dry air may be prevented from directly contacting the suspension tool by arranging an air flow direction-altering plate on the straight line interconnecting the inlet port and the suspension tool. Further, even in the case in which the direction of inflow of the dry air matches the direction of the straight line interconnecting the inlet port and the outlet port, for example, the air flow direction-altering plate may be arranged on the straight line interconnecting the inlet port and the outlet port, and the dry air may be made to pass through the vicinity of the through hole through which the suspension tool passes.

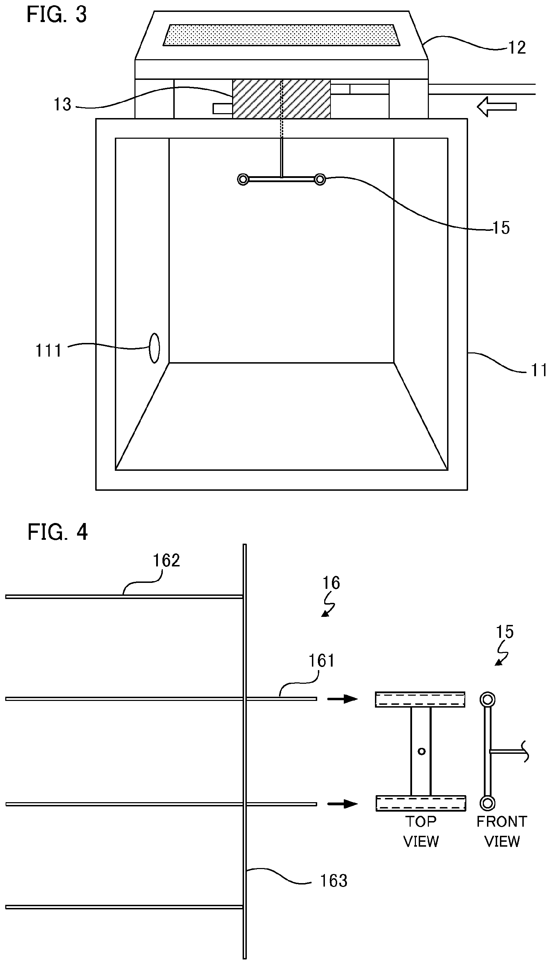

FIG. 3 is a front view illustrating the interior of the thermostatic chamber according to the embodiment. FIG. 3 illustrates a state in which the door of the thermostatic chamber 11 is removed. The suspension tool 15 is suspended in the interior of the thermostatic chamber 11 from the weighing pan supporter of the balance 12, through the ceiling and bottom through holes of the cover box 13, and through the through hole of the ceiling of the thermostatic chamber 11. Humidity of air is adjusted by the humidity generator 3, the humidity-adjusted air passes through the condensation-preventing heat-retention tube 4, and the humidity-adjusted air is supplied to the interior of the thermostatic chamber 11 from a supply port 111.

FIG. 4 is a drawing illustrating an example of a measurement tool according to the embodiment. As illustrated in FIG. 3 and FIG. 4, at ends positioned in the interior of the thermostatic chamber 11, the suspension tool 15 is equipped with two horizontally-supported tubes. The holder 16 includes a base 163 extending linearly, four tines 162 extending orthogonally from the base 163, and two beams 161 extending from the base 163 from the side opposite to the tines 162. Further, the number of the tines 162 may be greater than or equal to two, and the number of the beams 161 may be greater than or equal to two. Further, the number of tubes of the suspension tool 15 may be any number as long as the number is the same as the number of the beams 161.

The sample is fixed beforehand by penetration by the tines 162, and the beams 161 of the holder 16 are inserted into the tubes of the suspension tool 15, thereby enabling the sample in a single operation with respect to the suspension tool 15 to be positioned in the interior of the thermostatic chamber 11. This procedure enables a lowering of measurement error generated by the operation of the user arranging the sample in the interior of the thermostatic chamber 11. In order to further lower the measurement error, the measurement tool may be constructed from a material that is electrically conductive, non-magnetic, and non-hygroscopic. Further, the shape of the measurement tool is not limited to the shape of this example, and the shape may be any shape that does not contact the interior walls of the thermostatic chamber 11, and that makes possible suspension of the sample without contact with the interior walls of the thermostatic chamber 11.

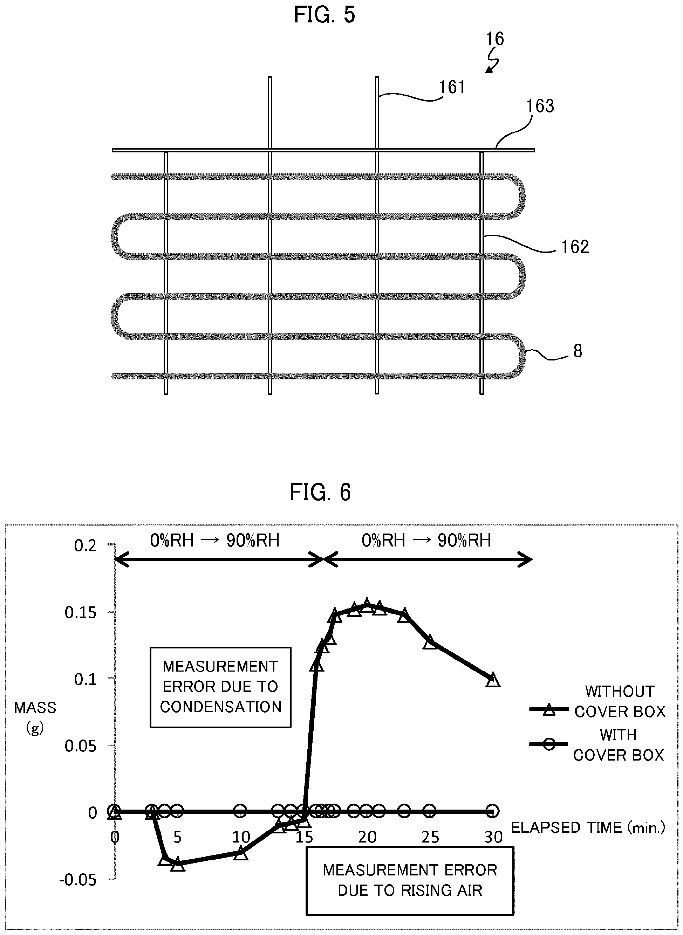

FIG. 5 is a top view illustrating an example of the holder fixing a sample according to the embodiment. When the sample 8 is cloth-like, and when the sample is folded and fixed so that gaps are formed between folds of the sample 8, a maximum amount of the sample 8 can be arranged in the interior of the thermostatic chamber 11 without contact between folds of the sample 8. This configuration increases the surface area of the sample 8 in contact with air, facilitating the measurement of the change of moisture absorption-desorption amount.

FIG. 6 is a graph illustrating results of weighing with and without providing of the cover box according to the embodiment. FIG. 6 is a graph of: results of measurement in a case in which a countermeasure was taken to prevent condensation by providing the cover box 13 was provided so that air within the thermostatic chamber 11 was prevented from rising toward the balance 12; and results of measurement in a case in which the cover box 13 was not provided, and no such countermeasure was taken. This graph indicates the change of mass due to the change of humidity in the state in which only the suspension tool 15 and the holder 16 were suspended from the balance within the thermostatic chamber 11, and the sample was not loaded. The vertical axis indicates the change of mass (g), and the horizontal axis indicates elapsed time (minutes).

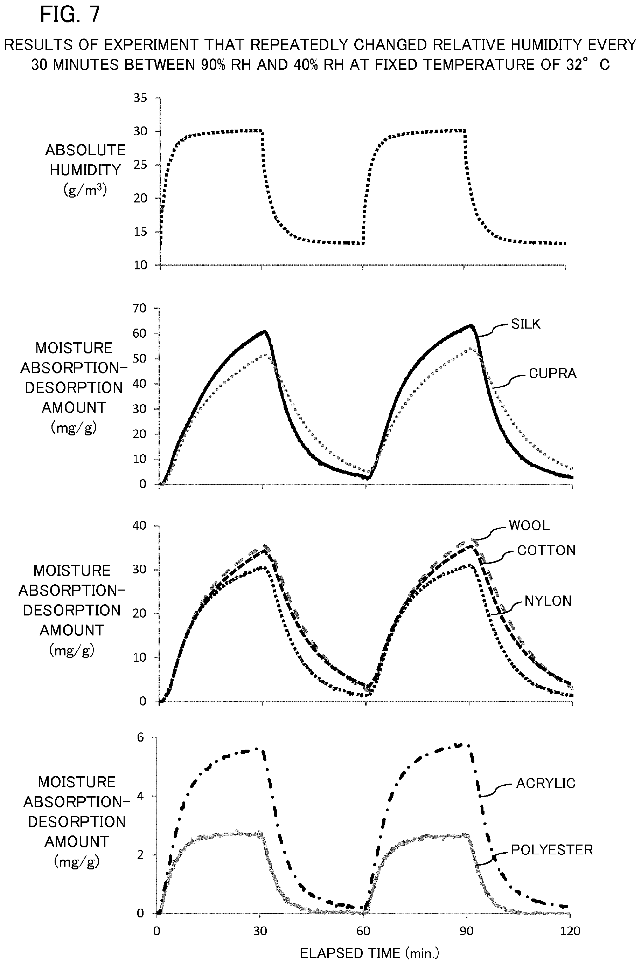

In the case in which the cover box 13 was provided, no sample was present in the interior of the thermostatic chamber 11, and thus the measured mass was nearly unchanged even after the humidity increased. However, in the case in which the cover box 13 was not provided, as illustrated in the graph, measurement error occurred due to condensation-induced measurement error, and due to air within the thermostatic chamber 11 rising toward the balance 12. Thus unless countermeasures are taken to prevent condensation by providing the cover box 13 to prevent the air within the thermostatic chamber 11 from rising toward the balance 12, the change of the moisture absorption-desorption amount cannot be measured. FIG. 6 illustrates results of measurement when a precision humidity generator manufactured by Daiichi-Kagaku Inc. was used as the humidity generator 3. The results illustrated in FIG. 7 to FIG. 9 were produced in the same manner.

FIG. 7 in graphs illustrates tracking ability and reproducibility of results of measurement, with respect to changes of humidity, of moisture absorption and desorption amounts measured by the humidity-dependent-mass measurement device according to the embodiment. FIG. 7 illustrates the measurement results of moisture desorption amounts for samples of the major fibers that are silk, wool, cotton, nylon, cupra, polyester, and acrylic. These graphs indicate the change of mass due to change of humidity in the state in which the sample was suspended from the balance 12 using the suspension tool 15 and the holder 16 within the thermostatic chamber 11. The vertical axis indicates the change of mass, that is, the moisture desorption amount (mg/g), relative to the sample mass when the sample is completely desiccated, and the horizontal axis indicates the elapsed time (minutes).

As illustrated in FIG. 7, when the absolute humidity within the thermostatic chamber 11 was switched between 13.5 g/m³ and 30.5 g/m³, the moisture desorption amount changed with the change of humidity. In this manner, the measurement results of the moisture absorption-desorption amount measured by the humidity-dependent-mass measurement device 1 could track the change of humidity. Further, as illustrated in FIG. 7, when the absolute humidity within the thermostatic chamber 11 was switched between 13.5 g/m³ and 30.5 g/m³, the moisture absorption-desorption amounts of each type of fiber sample changed similarly every 30 minutes. In this manner, the measurement results of the moisture absorption-desorption amount measured by the humidity-dependent-mass measurement device 1 were reproducible during test-retesting.

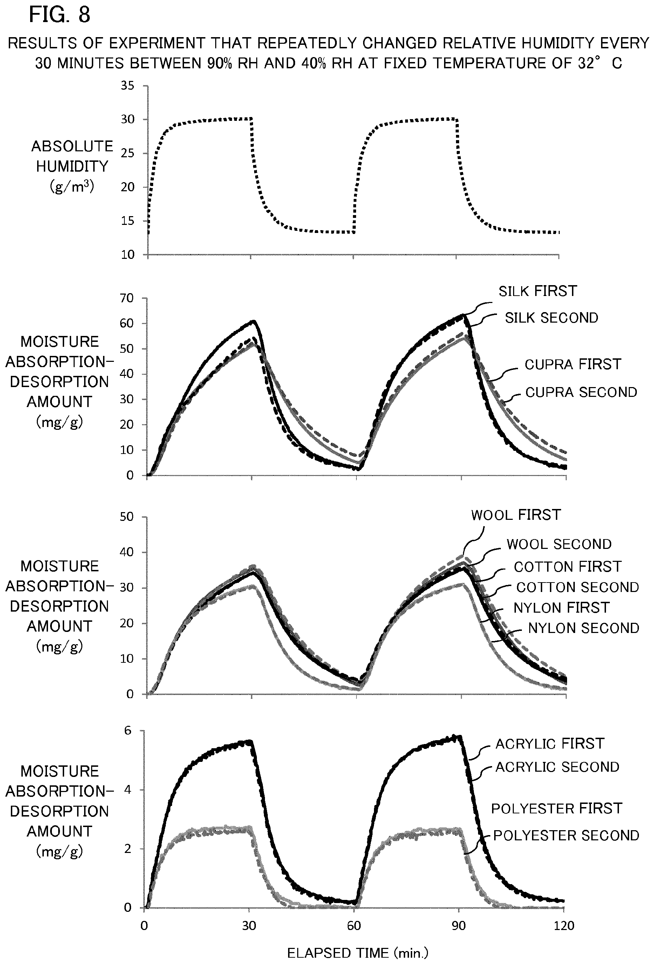

FIG. 8 in graphs illustrates reproducibility of results of measurement of the moisture absorption and desorption amounts measured by the humidity-dependent-mass measurement device according to the embodiment. FIG. 8 illustrates in graphs the first and second measurement results for the moisture desorption amounts for samples of the major fibers that are silk, wool, cotton, nylon, cupra, polyester, and acrylic. As illustrated in FIG. 8, the measurement results for the first and the second measurements were nearly the same. In this manner, the measurement results of the moisture absorption-desorption amount measured by the humidity-dependent-mass measurement device 1 were reproducible during test-retesting.

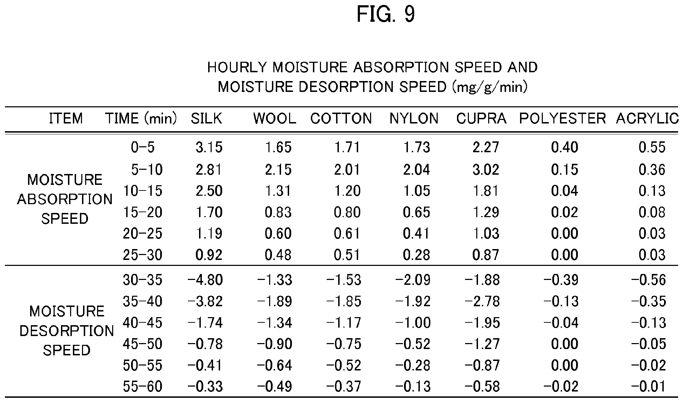

FIG. 9 is a table illustrating time-wise changes of a moisture absorption speed and a moisture desorption speed according to the embodiment. FIG. 9 is a table illustrating time-wise change of the moisture absorption speed and the moisture desorption speed calculated from the measurement results of the moisture desorption amounts of the fiber samples illustrated in FIG. 7. In this manner, the humidity-dependent-mass measurement device 1 enables the obtaining of the moisture absorption speed and the moisture desorption speed of the sample.

The humidity-dependent-mass measurement device 1 according to the present embodiment makes possible, in a manner that enables analysis of the speed of moisture adsorption and desorption of the sample, the measurement of mass of the sample for which the mass changes in accordance with humidity of the atmosphere. This enables measurement of the moisture absorption speed or the moisture desorption speed, which is an important factor in comfort when wearing clothing. Further, temperature and humidity changes in a short time period in day-to-day life, and thus the moisture desorption amount can be measured in a state that is nearer to the state of the environment in day-to-day life.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

### Reference Signs List

- 1: Humidity-dependent-mass measurement device
- 2: Dryer
- 3: Humidity generator
- 4: Condensation-preventing heat-retention tube
- 5: Thermostatic device
- 8: Sample
- 11: Thermostatic chamber
- 12: Balance
- 13: Cover box
- 14: Dry air supplier
- 15: Suspension tool
- 16: Holder
- 111: Supply port
- 161: Beam
- 162: Tine
- 163: Base

## Claims

1. A humidity-dependent-mass measurement device comprising:
a thermostatic chamber, defining a space in an interior thereof for containing a sample and including a ceiling having a through hole formed therein communicating between the space of the interior and an exterior of the thermostatic chamber, for maintaining an atmosphere of the interior at a determined temperature and humidity;
a balance supported above the thermostatic chamber;
a measurement tool for suspension from a weighing pan supporter of the balance, insertion through the through hole of the thermostatic chamber without contacting an inner circumferential surface of the through hole, and holding the sample in the interior of the thermostatic chamber;
a cover box including an air inlet port and an air outlet port in a side surface, the cover box containing therein a portion of the measurement tool between the thermostatic chamber and the balance in a state suspending the measurement tool from the weighing pan supporter without contacting the cover box; and
a dry air supplier for causing dry air at or below a specified humidity to flow into the cover box from the inlet port.

2. The humidity-dependent-mass measurement device according to claim 1, wherein a direction of the dry air flowing into the cover box from the inlet port is displaced from:
a direction of a straight line interconnecting the inlet port and the measurement tool, and
a direction of a straight line interconnecting the inlet port and the outlet port.

3. The humidity-dependent-mass measurement device according to claim 1 or 2, wherein
the inlet port of the cover box is formed in a first side surface of the cover box above a center of the first side surface, and
the outlet port is formed diagonally opposing the inlet port and in a second side surface opposing the first side surface, the outlet port being formed below a center of the second side surface.

4. The humidity-dependent-mass measurement device according to any one of claims 1 to 3, wherein the measurement tool comprises:
a suspension tool for suspension from the weighing pan supporter of the balance and insertion through the through hole of the thermostatic chamber; and
a holder, detachably attached to the suspension tool in the interior of the thermostatic chamber, for holding the sample.

5. The humidity-dependent-mass measurement device according to claim 4, wherein the holder of the measurement tool comprises a tine for penetrating the sample.

6. The humidity-dependent-mass measurement device according to claim 5, wherein:
the tine is a plurality of tines;
the holder of the measurement tool comprises: (i) a linearly extending base, (ii) the plurality of tines extending orthogonally from the base, and (iii) at least two beams extending from the base at a side of the base opposite to the plurality of tines; and
the suspension tool comprises in a lower portion thereof at least two horizontally supported tubes, each horizontally supported tube of the at least two horizontally supported tubes being for supporting a respective beam of the at least two beams.

7. The humidity-dependent-mass measurement device according to any one of claims 1 to 6, wherein the measurement tool is formed from a material that is electrically conductive, non-magnetic, and non-hydroscopic.

8. A humidity-dependent-mass measurement method using a humidity-dependent-mass measurement device including: (i) a thermostatic chamber, defining a space in an interior thereof for containing a sample and including a ceiling having a through hole formed therein communicating between the space of the interior and an exterior of the thermostatic chamber, for maintaining an atmosphere of the interior at a determined temperature and humidity, (ii) a balance supported above the thermostatic chamber, (iii) a measurement tool for suspension from a weighing pan supporter of the balance, insertion through the through hole of the thermostatic chamber without contacting an inner circumferential surface of the through hole, and holding the sample in the interior of the thermostatic chamber, (iv) a cover box including an air inlet port and an air outlet port in a side surface, the cover box containing therein a portion of the measurement tool between the thermostatic chamber and the balance in a state suspending the measurement tool from the weighing pan supporter without contacting the cover box, and (v) a dry air supplier for causing dry air at or below a specified humidity to flow into the cover box from the inlet port, the method comprising:
causing the sample to be held by the measurement tool within the interior of the thermostatic chamber;
maintaining the atmosphere of the interior of the thermostatic chamber at a determined temperature and a determined humidity; and
measuring a mass of the sample by the balance in a state in which the dry air at or below the specified humidity is made to flow from the inlet port of the cover box into the cover box.
